# FASCICULE DE BREVET EUROPEEN

(11) **EP 4 101 331 B1**
(45) Date de publication et mention de la délivrance du brevet: **27.03.2024**
(21) Numéro de dépôt: 22020263.4
(22) Date de dépôt: 04.06.2022
(51) Int. Cl.: A43B 7/20, A61F 5/01

(54) **DISPOSITIF DE PROTECTION DE CHEVILLE**
KNÖCHELSCHUTZ
ANKLE PROTECTION DEVICE

(30) Priorité: 08.06.2021 FR 2106053
(43) Date de publication de la demande: 14.12.2022
(73) Titulaire: de Lafaye-Cariven, Maxime, 83740 La Cadiere d'Azur (FR)
(72) Inventeur: de Lafaye-Cariven, Maxime, 83740 La Cadiere d'Azur (FR)

(56) Documents cités:
- WO-A1-00/18348
- WO-A1-2014/176368

## Description

### Domaine technique

La présente invention se rapporte à un dispositif de protection de cheville d'une personne, plus précisément des malléoles. Ce type de dispositif peut être utilisé principalement par les usagers de véhicules motorisés tels que la moto, le cyclomoteur, la mobylette, le scooter, les trois-roues voire même de quads.

### Arrière-Plan Technologique

Les chevilles et particulièrement les malléoles d'usagers de véhicules deux-roues et/ ou trois-roues motorisés sont des organes souvent touchés lors d'un choc voire même d'une chute. En effet, les malléoles, terme désignant l'une des deux tubérosités osseuses situées au niveau de l'articulation de la cheville d'une personne, apparaissant sous la forme d'une saillie sous la peau de chaque côté de celle-ci, peuvent être sujettes à l'abrasion avec un écorchement à vif, à des fêlures voire parfois à des fractures. Ainsi, la protection de ces organes et donc l'utilisation d'équipement de protection de la cheville et plus précisément des malléoles est une nécessité.

Actuellement, les principaux équipements de protection des chevilles connus, notamment pour motards, se trouvent être des bottes et/ou des chaussures de moto. De tels équipements possèdent une certaine rigidité et présentent plusieurs renforts disposés au niveau des points sensibles tels que, par exemple les malléoles. Pour des questions de confort et/ou d'esthétisme, certains usagers ne souhaitent pas porter de tels équipements en dehors de leur trajet motorisé. De telles chaussures et/ou bottes de moto sont souvent lourdes, imposantes et pas toujours au goût de l'usager notamment selon sa tenue vestimentaire. Or, de nombreux usagers privilégient avant tout le style, le confort et la facilité de porter des chaussures de ville que ce soit pour leur journée de travail et/ou dans le cas de leurs loisirs. Ainsi, cela implique que l'usager prévoit d'emporter avec lui une paire de chaussures de ville en rechange et dispose d'un conditionnement ou d'un stockage adéquat pour ses bottes et/ou chaussures de moto. Or, le transport et le stockage de chaussures de rechange et de bottes et/ou de chaussures de moto n'étant pas pratique, il est souvent fait le choix de ne pas porter de chaussures ou de bottes de moto adaptées car considérées trop souvent comme encombrantes, ce qui est un choix déraisonnable au regard des risques encourus.

WO 2014/176368 A1 divulgue un dispositif de protection de cheville selon l'art antérieur.

### Résumé de l'invention

La présente invention vise donc à remédier aux inconvénients précités, notamment à proposer un dispositif de protection de cheville et plus précisément des malléoles contre les chocs et l'abrasion pouvant résulter de chutes à moto par exemple. Ledit dispositif est conçu pour suppléer l'absence de protection des chaussures de ville tout en étant compact et donc facile à ranger dans un sac ou une poche, par exemple. Ainsi, l'usager a la possibilité de garder ses chaussures de ville pendant sa journée et vient simplement rajouter ledit dispositif de protection sur ses chevilles lorsqu'il souhaite se déplacer avec son véhicule motorisé.

À cet effet, l'invention divulgue un dispositif de protection de cheville d'une personne selon la revendication 1. Ledit dispositif comporte une pièce principale, deux renforts, des moyens de fermeture et un élément rigide. Ladite pièce principale est composée d'un matériau anti-abrasion enveloppant la cheville et dispose d'une partie arrière placée sur l'arrière de la cheville, au niveau du tendon d'Achille de la personne, d'une partie avant placée sur l'avant de la cheville, au niveau du cou-de-pied de la personne, d'une paroi intérieure destinée à être au contact de la cheville et d'une paroi extérieure. Les deux renforts sont fixés à la paroi intérieure de ladite pièce principale au niveau des malléoles de la cheville de la personne. Les moyens de fermeture sont fixés à la paroi extérieure de la pièce principale permettant de maintenir et serrer ledit dispositif contre la cheville. L'élément rigide présente deux parties reliées entre elles par un coude, une partie supérieure étant fixée à la partie arrière de la pièce principale et une partie inférieure étant destinée à être positionnée sous le talon d'une chaussure de la personne.

Selon un mode de réalisation privilégié, le matériau constitutif de la pièce principale peut être un cuir gras.

Selon un mode de réalisation préféré, les moyens de fermeture peuvent correspondre à des sangles et des boucles fixées de part et d'autre de la paroi extérieure de la pièce principale.

Préférentiellement, les sangles peuvent être principalement constituées de cuir et peuvent comporter une zone en matière auto-agrippante.

De préférence, les renforts sont constitués de polyuréthane thermoplastique, d'acrylonitrile butadiène styrène ou de polypropylène.

Afin d'apporter une rigidité supplémentaire, ledit dispositif peut comporter en outre une plaque de renfort haute densité fixée sur la paroi intérieure de la pièce principale permettant de maintenir en position les deux renforts sur la paroi intérieure de la pièce principale.

Afin de faciliter l'adaptation dudit dispositif à différentes morphologies, l'élément rigide peut être fixé à la partie arrière de la pièce principale par l'intermédiaire de moyens de fixation ajustables en hauteur. Les moyens de fixation ajustables en hauteur peuvent comporter un premier élément localisé sur la partie supérieure de l'élément rigide et un deuxième élément localisé sur la partie arrière de la pièce principale. Lesdits premier et deuxième éléments vont ainsi coopérer l'un avec l'autre pour maintenir le premier élément sur le deuxième élément selon plusieurs positions.

De préférence, le premier élément est un trou oblong et le deuxième élément consiste en des inserts filetés maintenus à la partie arrière de la pièce principale par la pièce de renfort haute densité afin de recevoir des vis. Le trou oblong, quant à lui, permet de laisser passer les vis et d'assurer un ajustement à la hauteur souhaitée.

Afin d'apporter un confort et une adhésion supplémentaires, ledit dispositif de protection d'une cheville de la personne peut comporter en outre une mousse positionnée sur la paroi intérieure de ladite pièce principale. Ladite mousse vient épouser la forme de la cheville de la personne.

En complément, ledit dispositif peut comporter en outre une chaussette enveloppant l'avant du pied. Ladite chaussette et la pièce principale sont munies chacune de boutons pression afin de fixer ladite chaussette audit dispositif. Les boutons pression de ladite chaussette sont destinés à coopérer avec les boutons pression de la pièce principale.

### Brève Description des figures

L'invention sera mieux comprise et d'autres caractéristiques et avantages de celle-ci apparaîtront à la lecture de la description suivante d'un mode de réalisation particulier de l'invention, donné à titre d'exemple illustratif et non limitatif, et faisant référence aux dessins annexés, parmi lesquels :
[Fig.1] montre une vue de profil d'un exemple de réalisation d'un dispositif de protection de cheville droite d'une personne selon l'invention,
[Fig.2] montre une vue arrière du dispositif de protection de cheville mis à plat présentant des détails intermédiaires de fabrication,
[Fig.3] montre une vue intérieure du dispositif de protection de cheville mis à plat présentant des détails intermédiaires de fabrication,
[Fig.4] montre une vue intérieure du dispositif de protection de cheville d'une personne selon une variante de réalisation,
[Fig.5] montre une vue arrière du dispositif de protection de cheville mis à plat présentant des détails intermédiaires de fabrication,
[Fig.6] montre une vue arrière du dispositif mis à plat de la [Fig.1],
[Fig.7] montre une vue de profil d'un exemple de réalisation d'un dispositif de protection de cheville gauche selon une variante de réalisation.

### Description détaillée

Afin de simplifier la description, une même référence est utilisée dans différentes figures pour désigner un même objet. Ainsi, lorsque la description cite un objet référencé, cet objet pourra être identifié sur plusieurs figures. En outre, les figures ainsi que la description sont données à titre d'exemples non limitatifs de réalisation. De plus, à des fins illustratives, le dispositif de protection de cheville d'une personne, selon l'invention, va être décrit de manière plus détaillée, dans la suite de la description, pour une utilisation par un usager de véhicules deux-roues et/ou trois-roues motorisés.

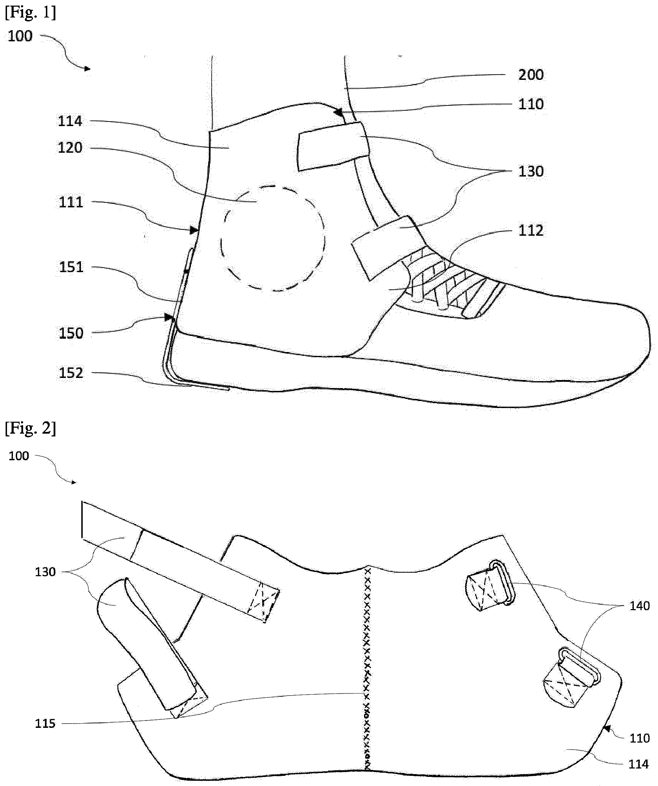

Un exemple de dispositif de protection de cheville 100 d'une personne, conforme à l'invention, est représenté sur la [Fig.1]. Ledit dispositif de protection de cheville 100 est composé d'une pièce principale 110 enveloppant la cheville 200 de la personne, de deux renforts 120 fixés à la pièce principale 110, de moyens de fermeture permettant de serrer ledit dispositif 100 contre la cheville 200. De plus, afin de permettre un bon maintien dudit dispositif 100 contre la cheville 200 et donc d'assurer une protection efficace des malléoles, ledit dispositif 100, selon l'invention, comporte un élément rigide 150 fixé à la pièce principale 110 et prenant appui sous le talon d'une chaussure de la personne.

La pièce principale 110 est dimensionnée pour venir se positionner au-dessus de la chaussure de la personne afin d'envelopper la cheville 200. Pour ce faire, elle dispose d'une partie arrière 111 qui vient se placer sur l'arrière de la cheville 200 localisé au niveau du tendon d'Achille de la personne et d'une partie avant 112 qui vient se placer sur l'avant de la cheville 200 localisé au niveau du cou-de-pied de la personne. De plus, ladite pièce principale 110 présente une paroi intérieure 113 qui est destinée à venir au contact de la cheville 200 de la personne et une paroi extérieure 114 qui est à l'opposé de la paroi intérieure 113 et donc potentiellement sujette aux agressions extérieures telles que l'abrasion, les chocs et les impacts.

Ainsi, pour apporter une protection suffisante face aux agressions extérieures notamment lors de chutes, ladite pièce principale 110 est composée d'un matériau anti-abrasion. Dans un mode de réalisation préféré, un tel matériau anti-abrasion peut être un cuir gras. En effet, le cuir gras est un cuir particulièrement robuste et très adapté aux conditions d'utilisation difficiles. À ce titre, il est aujourd'hui très souvent utilisé pour des chaussures techniques de travail et/ou de randonnée par exemple. Dans le cas de l'invention, il est privilégié un cuir gras entre 1,6 et 2,2 millimètres d'épaisseur, préférentiellement 2 millimètres, afin de disposer d'une résistance anti-abrasion suffisante pour la protection recherchée et d'une élasticité avantageuse pour pouvoir travailler le cuir et permettre la fabrication de ladite pièce principale 110. Néanmoins, l'invention ne se limite pas à ce choix de matériau de ladite pièce principale, l'homme du métier pourra utiliser tout autre matériau compatible avec l'utilisation qui en est faite au sein de l'invention, à savoir un matériau alliant une résistance à l'abrasion et une certaine élasticité permettant une mise en forme aisée. À titre d'exemple, il peut être utilisé des tissus techniques tels que des tissus à base d'aramide ou des tissus combinant nylon et élasthanne ou encore des textiles à base de polyamide.

Tel qu'illustrée en [Fig.2], une telle pièce principale 110 peut être formée par deux parties cousues entre elles. Dans ce cas, la zone de couture 115 est localisée au niveau de la partie arrière 111 de ladite pièce principale 110 afin que ladite partie arrière 111 corresponde et épouse le galbe de l'arrière de la cheville 200 de la personne. La couture 115 apparente sur la paroi extérieure 114 peut être recouverte, d'une bande cousue sur toute la longueur de la partie arrière 111 et de part et d'autre de ladite couture 115 afin de cacher ladite couture 115 pour des considérations esthétiques mais également afin de la consolider. Cette bande cousue peut être en sergé, ce qui permet de renforcer la résistance à l'usure. En variante, la pièce principale 110 peut être réalisée en une seule partie qui est mise en forme par des procédés de formage dépendants du matériau constituant la pièce principale 110.

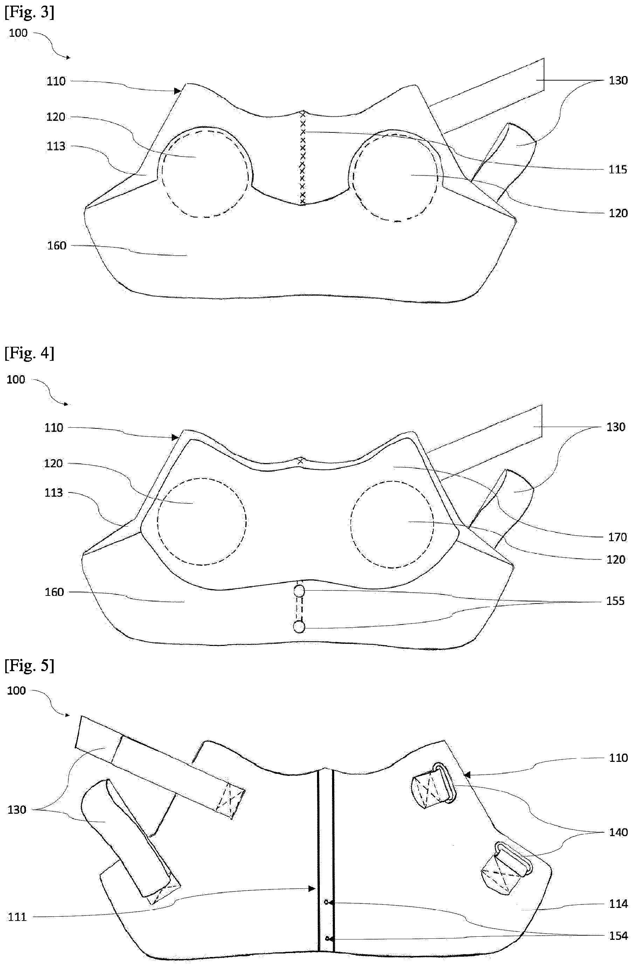

Tel qu'illustré en [Fig.3], les deux renforts 120 sont fixés à la paroi intérieure 113 de ladite pièce principale au niveau de chaque malléole de la cheville 200 de la personne afin d'en assurer la protection lors de chocs et de réduire l'échauffement dû à l'abrasion. Les deux renforts 120 peuvent être fixés à la paroi intérieure 113 par couture, par insertion dans des poches prévues à cet effet ou tout autre moyen permettant le maintien desdits renforts 120 au sein dudit dispositif 100 et au niveau de chaque malléole. De tels renforts 120 peuvent être des coques concaves afin que lesdits renforts 120 englobent correctement chacune des malléoles de la personne. Lesdites coques seront dimensionnées de façon à ce que les renforts 120 puissent s'adapter à un très grand nombre de malléole indépendamment de la corpulence de la personne. Par exemple, il peut être envisagé d'utiliser des disques incurvés de 70 à 80 millimètres de diamètre sur 1,5 millimètres d'épaisseur avec une incurvation de 10 millimètres. Par ailleurs, afin d'apporter la protection suffisante contre des chocs, de tels renforts 120 peuvent être constitués, par exemple, de polyuréthane thermoplastique, classiquement utilisé comme matériau dans les bottes de moto. Néanmoins, l'invention ne se limite pas à ce choix de matériau pour les renforts 120, l'homme du métier pourra utiliser tout autre matériau compatible avec l'utilisation qui en est faite au sein de l'invention. À titre d'exemple, il est également possible d'utiliser des renforts à base d'acrylonitrile butadiène styrène (ABS), à base de polypropylène ou encore à base de fluide non-newtonien rhéoépaississant type D30^{®}.

En complément, afin d'apporter une rigidité supplémentaire audit dispositif de protection de cheville d'une personne 100, ledit dispositif 100 peut comporter en outre une plaque de renfort haute densité 160. Comme cela est visible sur la [Fig.3], cette plaque de renfort haute densité 160 est fixée, par exemple cousue, à la paroi intérieure 113 de ladite pièce principale 110. Ainsi, lesdits renforts 120 fixés à la paroi intérieure 113 peuvent être fixés à l'aide de ladite plaque de renfort 160, ce qui permet de maintenir lesdits renforts 120 en position au niveau de chacune des malléoles. À ce titre, les renforts 120 peuvent être enserrés entre la paroi intérieure 113 de la pièce principale 111 et ladite plaque de renfort haute densité 160. Lesdits renforts 120 peuvent même, par exemple, être cousus à ladite plaque de renfort haute densité 160 ou insérés dans ladite plaque de renfort haute densité 160. Afin d'apporter la rigidité suffisante, une telle plaque de renfort haute densité 160 peut être du Texon^{®}, consistant en des matières fibreuses non tissées liées par un élastomère, présentant préférentiellement une épaisseur comprise entre 1 et 1,5 millimètres. Néanmoins, l'invention ne se limite pas au choix du matériau de ladite plaque de renfort 160 ni même à son épaisseur.

Selon un mode de réalisation privilégié, tel qu'illustré sur la [Fig.2], les moyens de fermeture peuvent être des sangles 130 et des boucles 140 fixées de part et d'autre de la paroi extérieure 114 de ladite pièce principale 110. Chaque sangle 130 est destinée à coopérer avec une boucle 140 afin de maintenir et serrer le dispositif 100 contre la cheville 200. À titre d'exemple, ledit dispositif 100 peut comporter deux paires de sangles 130 et de boucles 140, une paire pouvant être positionnée au niveau du tibia et une autre paire au niveau du cou-de-pied. Quant à la fixation des sangles 130 et/ou des boucles 140 à la paroi extérieure 140, il est possible d'utiliser un système de fixation par couture, par collage ou même par vissage, l'invention ne se limitant pas au choix du système de fixation des sangles 130 et boucles 140.

Dans un mode de réalisation préféré, les sangles 130 correspondent à des lanières en cuir ou en matériau anti-abrasion, présentant une zone en matière auto-agrippante type Velcro^{®} et les boucles 140 correspondent à un anneau, un rectangle ou autre, présentant une traverse destinée à recevoir une ou des lanières. Chaque lanière, correspondante à une des sangles 130, est ainsi introduite dans la traverse d'une des boucles 140 et vient se rabattre sur elle-même : l'adhésion étant assurée par la zone auto-agrippante de la sangle 130. Lesdites lanières seront dimensionnées de façon à ce que ledit dispositif 100 puisse s'adapter à un large panel de cheville. Par exemple, il peut être envisagé d'utiliser des lanières de 200 millimètres de longueur sur 25 millimètres de large. En variante, tout autre moyen de fermeture compatible avec l'utilisation qui en est faite dans l'invention, tels que des moyens de fermeture par clipsage ou à lacet voire même proches de ceux utilisés pour des chaussures de ski, peut être utilisé en lieu et place des sangles 130 et boucles 140.

Tel qu'illustré sur la [Fig.1], l'élément rigide 150 est fixé à la paroi extérieure 114 de la pièce principale 110 et vient se placer sous le talon de la chaussure de la personne. Pour ce faire, ledit élément rigide 150 présente une partie supérieure 151 fixée à la partie arrière 111 de la pièce principale 110 et une partie inférieure 152 destinée à être positionnée sous le talon de la chaussure de la personne. La partie supérieure 151, présentant une direction sensiblement verticale, est reliée par un coude à la partie inférieure 152, présentant une direction sensiblement horizontale. À titre d'exemple, l'élément rigide 150 peut sensiblement être en forme de L. Pour le choix du matériau de l'élément rigide 150, il est privilégié un matériau présentant une bonne résistance aux agressions extérieures et une densité moyenne afin de ne pas alourdir ledit dispositif 100. Par exemple, l'élément rigide 150 peut être constitué d'acier inoxydable d'épaisseur de 1,5 millimètres. Néanmoins, l'invention ne se limite pas à ce choix de matériau pour l'élément rigide 150, l'homme du métier pourra utiliser tout autre matériau compatible avec l'utilisation qui en est faite au sein de l'invention. À titre d'exemple, il est également possible d'utiliser un élément rigide 150 en matériau composite.

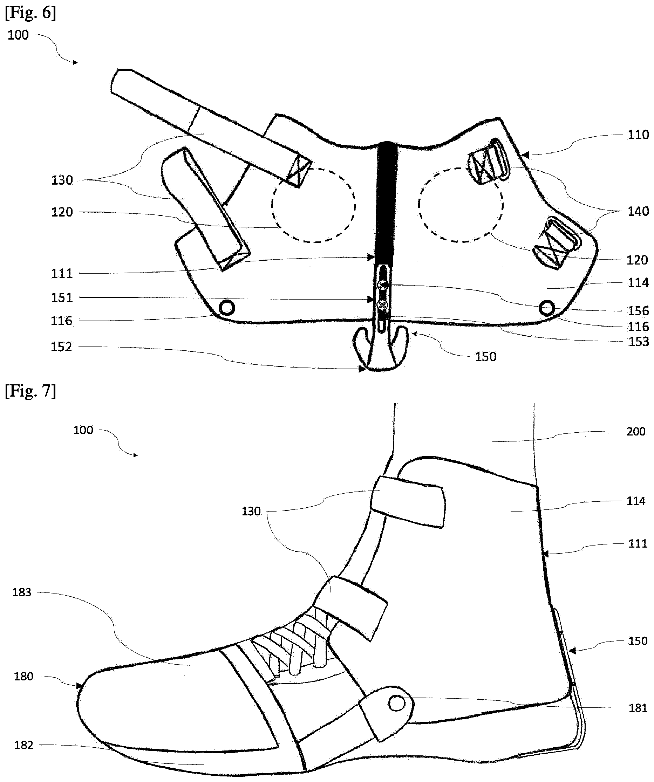

Selon un mode de réalisation préféré, afin que ledit dispositif 100 puisse s'adapter à différents types de chaussures et notamment puisse se régler selon l'épaisseur de la semelle de la chaussure, la partie supérieure 151 de l'élément rigide 150 peut être fixée à la partie arrière 111 de la pièce principale 110 par l'intermédiaire de moyens de fixation ajustables en hauteur. À ce titre, tel qu'illustré sur les figures 5 et 6, lesdits moyens de fixation ajustables en hauteur peuvent comporter un premier élément 153 localisé sur la partie supérieure 151 de l'élément rigide 150 et un deuxième élément 154 localisé sur la partie arrière 111 de la pièce principale 110. Les premier 153 et deuxième 154 éléments vont coopérer l'un avec l'autre pour maintenir le premier élément 153 sur le deuxième élément 154 selon plusieurs positions.

À titre d'exemple, le premier élément 153 peut correspondre à un trou oblong qui permet à l'utilisateur de régler l'élément rigide 150 à la hauteur souhaitée selon l'épaisseur de la semelle de sa chaussure. Le deuxième élément 154, quant à lui, peut correspondre à des inserts filetés. Pour ce faire, tel qu'illustré sur la [Fig.4], des perforations 155 sont réalisées dans la pièce de renfort haute densité 160 et dans la pièce principale 110 au niveau de la partie arrière 111. Ainsi, lesdits inserts filetés viennent se placer au sein des perforations 155, tel qu'illustré en [Fig.5]. À cet effet, lesdits inserts filetés sont maintenus à la partie arrière 111 de la pièce principale 110 à l'aide de la pièce de renfort haute densité 160. Les inserts filetés, correspondant au deuxième élément 154 et le trou oblong, correspondant au premier élément 153, sont respectivement dimensionnés pour recevoir et laisser passer des vis 156. Ainsi, une fois que l'utilisateur a fait coulisser la partie arrière 111 de la pièce principale 110 et donc les inserts filetés le long du trou oblong et a atteint la hauteur souhaitée, il lui suffit de solidariser les premier 153 et deuxième 154 éléments par des vis 156.

Selon une variante, la partie supérieure 151 de l'élément rigide 150 peut être fixée à la partie arrière 111 de la pièce principale 110 simplement par vissage ou par clipsage.

En complément, tel qu'illustré sur la [Fig.4], ledit dispositif 100 peut comporter en outre une mousse 170 positionnée sur la paroi intérieure 113 de ladite pièce principale 110. Afin d'apporter un confort et une adhésion supplémentaires à la personne, ladite mousse 170 vient épouser la forme de la cheville 200 de la personne. Préférentiellement, ladite mousse 170 est positionnée en partie haute dudit dispositif, à savoir au niveau du tendon d'Achille de la personne. Dans le cas où le dispositif 100 comporte la plaque de renfort haute densité 160, ladite mousse 170 peut venir se placer sur ladite plaque 160. En variante, ladite mousse 170 peut venir se placer directement sur la paroi intérieure 113 de ladite pièce principale 110 et les deux renforts 120. À ce titre, la mousse 170 peut venir remplacer la pièce de renfort haute densité 160. Il suffit seulement qu'une telle mousse 170 combine à la fois une haute densité et une rigidité suffisante.

Ladite mousse 170 peut être fixée audit dispositif 100 par couture, par collage ou autre. Une telle mousse 170 permet notamment d'absorber les chocs et de réduire les effets de frottement pouvant générer des brûlures sur la cheville 200 de la personne. À titre d'exemple, la mousse 170 peut être constituée d'un caoutchouc synthétique type EPDM, abréviation d'éthylène, propylène, diène et monomère, préférentiellement avec une épaisseur de 5 millimètres et une densité de l'ordre de 140 kilogrammes par mètre cube. Néanmoins, l'invention ne se limite pas au choix du matériau pour la mousse 170 ni même à son épaisseur et à sa densité, l'homme du métier pourra utiliser tout autre matériau compatible avec l'utilisation qui en est faite au sein de l'invention, à savoir un matériau absorbant les chocs et apportant un confort et une adhésion suffisantes.

Selon un mode de réalisation visant à protéger la chaussure de la personne de l'usure due à des contacts répétés de ladite chaussure avec le sélecteur de vitesse du véhicule motorisé, ledit dispositif de protection de cheville d'une personne 100 peut comporter en outre une chaussette 180 enveloppant l'avant du pied. Ladite chaussette est fixée audit dispositif 100 par l'intermédiaire de boutons pressions 116 et 181. À cet effet, la pièce principale 110 comporte sur sa paroi extérieure 114 de part et d'autre en partie basse dudit dispositif, au niveau du cou-de-pied de la personne, des boutons pressions 116, tel qu'illustré sur la [Fig.6]. Parallèlement, comme le montre la [Fig.7], ladite chaussette 180 comporte de chaque côté un bouton pression 181 destiné à coopérer avec chaque bouton pression de la pièce principale 116. Afin de disposer d'une élasticité suffisante permettant d'enfiler facilement la chaussette 180 sur l'avant du pied de la personne tout en apportant le niveau de résistance à l'usure sans limiter la tenue à l'abrasion dudit dispositif 100, une telle chaussette 180 peut être principalement constituée d'une première pièce 182 en tissu souple, type jersey, recouverte sur le dessus d'une deuxième pièce 183 en matériau anti-abrasion, type cuir gras ou autre précédemment décrit. Préférentiellement, la chaussette 180 peut être ajourée au niveau de la partie en contact avec le cale-pied du véhicule motorisé afin de favoriser l'appui. En variante, il est possible d'ajouter une semelle en dessous de la chaussette 180, correspondant à la partie en contact avec le sol, telle que la pointe du pied, lorsque l'usager pose le pied à terre. Cela permet ainsi d'éviter également une usure précoce de la chaussette 180 à la suite de contacts répétés avec le sol.

Il sera apprécié de l'homme du métier que la présente divulgation n'est pas limitée à ce qui est particulièrement montré et décrit ci-dessus. D'autres modifications peuvent être envisagées sans sortir du cadre de la présente invention défini par les revendications ci-annexées. Notamment, dans l'exemple préféré décrit précédemment, les principaux composants du dispositif de protection de cheville d'une personne 100 conforme à l'invention ont été décrits. Pour des besoins esthétiques et/ou de confort, il est possible d'imaginer que ledit dispositif 100 peut comporter des composants complémentaires et optionnels.

À titre d'exemple, il est possible d'avoir une pièce supplémentaire, cousue sur la paroi intérieure 113 de ladite pièce principale 110 et en contact direct avec la cheville 200 de la personne : cette pièce correspondant à une doublure intérieure dudit dispositif 100. Une telle pièce peut être en jersey coton, matière présentant l'avantage d'être un très bon isolant et d'apporter un effet thermorégulateur limitant les phénomènes de transpiration. De même, un morceau de tissu type feutrine, matière légère et présentant une respirabilité naturelle, pourra être cousue à la partie arrière 111 de la pièce principale 110 afin de recouvrir la base des inserts filetés évitant notamment une usure de la doublure intérieure.

## Revendications

1. Dispositif de protection de cheville d'une personne (100) comportant :
- une pièce principale (110) composée d'un matériau anti-abrasion enveloppant la cheville (200) et disposant d'une partie arrière (111) placée sur l'arrière de la cheville (200) au niveau du tendon d'Achille de la personne, d'une partie avant (112) placée sur l'avant de la cheville (200) au niveau du cou-de-pied de la personne, d'une paroi intérieure (113) destinée à être au contact de la cheville (200) et d'une paroi extérieure (114),
- deux renforts (120) fixés à la paroi intérieure (113) de la pièce principale (110) au niveau des malléoles de la cheville (200) de la personne,
- des moyens de fermeture fixés à la paroi extérieure (114) de la pièce principale (110) permettant de maintenir et serrer ledit dispositif (100) contre la cheville (200),
- un élément rigide (150) présentant deux parties reliées entre elles par un coude, une partie supérieure (151) fixée à la partie arrière (111) de la pièce principale (110) et une partie inférieure (152) destinée à être positionnée sous le talon d'une chaussure de la personne.

2. Dispositif de protection de cheville d'une personne (100) selon la revendication précédente, pour lequel le matériau constitutif de la pièce principale (110) est un cuir gras.

3. Dispositif de protection de cheville d'une personne (100) selon l'une des revendications précédentes, pour lequel les moyens de fermeture correspondent à des sangles (130) et des boucles (140) fixées de part et d'autre de la paroi extérieure (114) de la pièce principale (110).

4. Dispositif de protection de cheville d'une personne (100) selon la revendication précédente, pour lequel les sangles (130) sont principalement constituées de cuir et comportent une zone en matière auto-agrippante.

5. Dispositif de protection de cheville d'une personne (100) selon l'une des revendications précédentes, pour lequel les renforts (120) sont constitués de polyuréthane thermoplastique, d'acrylonitrile butadiène styrène ou de polypropylène.

6. Dispositif de protection de cheville d'une personne (100) selon l'une des revendications précédentes, comportant en outre une plaque de renfort haute densité (160) fixée sur la paroi intérieure (113) de la pièce principale (110) permettant de maintenir en position les deux renforts (120) sur la paroi intérieure (113) de la pièce principale (110).

7. Dispositif de protection de cheville d'une personne (100) selon l'une des revendications précédentes, pour lequel l'élément rigide (150) est fixé à la partie arrière (111) de la pièce principale (110) par l'intermédiaire de moyens de fixation ajustables en hauteur comportant un premier élément (153) localisé sur la partie supérieure (151) de l'élément rigide (150) et un deuxième élément (154) localisé sur la partie arrière (111) de la pièce principale (110), de sorte que lesdits premier (153) et deuxième (154) éléments vont coopérer l'un avec l'autre pour maintenir le premier élément (153) sur le deuxième élément (154) selon plusieurs positions.

8. Dispositif de protection de cheville d'une personne (100) selon les revendications 6 et 7, pour lequel le premier élément (153) est un trou oblong et le deuxième élément (154) consiste en des inserts filetés maintenus à la partie arrière (111) de la pièce principale (110) par la pièce de renfort haute densité (160) afin de recevoir des vis (156), le trou oblong permettant de laisser passer les vis (156) et d'assurer un ajustement à la hauteur souhaitée.

9. Dispositif de protection de cheville d'une personne (100) selon l'une des revendications précédentes, lequel comporte en outre une mousse (170) positionnée sur la paroi intérieure (113) de ladite pièce principale (110) venant épouser la forme de la cheville (200) de la personne.

10. Dispositif de protection de cheville d'une personne (100) selon l'une des revendications précédentes, comportant en outre une chaussette (180) enveloppant l'avant du pied, ladite chaussette (180) et la pièce principale (110) étant munies chacune de boutons pression (181, 116) afin de fixer ladite chaussette (180) audit dispositif (100), les boutons pression (181) de ladite chaussette (180) étant destinés à coopérer avec les boutons pression (116) de la pièce principale (110).

## Patentansprüche

1. Vorrichtung zum Schutz des Knöchels einer Person (100), **dadurch gekennzeichnet, dass** sie umfasst:
- einen Hauptteil (110), welcher aus einem abriebfesten Material besteht, welcher den Knöchel (200) umhüllt und über einen hinteren Teil (111), der auf der Rückseite des Knöchels (200) auf Höhe der Achillessehne der Person angeordnet ist, einen vorderen Teil (112), der auf der Vorderseite des Knöchels (200) auf Höhe des Rists der Person angeordnet ist, eine Innenwand (113), die dazu bestimmt ist, mit dem Knöchel (200) in Kontakt zu sein, und eine Außenwand (114) verfügt,
- zwei Verstärkungen (120), die an der Innenwand (113) des Hauptteils (110) in Höhe der Malleolus des Knöchels (200) der Person befestigt sind,
- Schließmittel, die an der Außenwand (114) des Hauptteils (110) befestigt sind und es ermöglichen, die genannte Vorrichtung (100) gegen den Dübel (200) zu halten und festzuklemmen,
- ein starres Element (150) welcher zwei Teile, die durch eine Krümmung miteinander verbunden sind, einen oberen Teil (151), der an dem hinteren Teil (111) des Hauptteils (110) befestigt ist, und einen unteren Teil (152), der dazu bestimmt ist, unter der Ferse eines Schuhs der Person positioniert zu werden, aufweist.

2. Vorrichtung (100) zum Schutz des Knöchels einer Person nach dem vorhergehenden Anspruch, bei der das Material, aus dem das Hauptteil (110) besteht, ein Fettleder ist.

3. Vorrichtung zum Schutz des Knöchels einer Person (100) nach einem der vorhergehenden Ansprüche, bei der die Verschlussmittel Riemen (130) und Schnallen (140) entsprechen, die auf beiden Seiten der Außenwand (114) des Hauptteils (110) befestigt sind.

4. Vorrichtung zum Schutz des Knöchels einer Person (100) nach dem vorhergehenden Anspruch, bei der die Riemen (130) hauptsächlich aus Leder bestehen und einen Bereich aus Klettmaterial aufweisen.

5. Vorrichtung zum Schutz des Knöchels einer Person (100) nach einem der vorhergehenden Ansprüche, bei der die Verstärkungen (120) aus thermoplastischem Polyurethan, Acrylnitril-Butadien-Styrol oder Polypropylen bestehen.

6. Vorrichtung zum Schutz des Knöchels einer Person (100) nach einem der vorhergehenden Ansprüche, die außerdem eine hochdichte Verstärkungsplatte (160) umfasst, die an der Innenwand (113) des Hauptteils (110) befestigt ist und es ermöglicht, die beiden Verstärkungen (120) an der Innenwand (113) des Hauptteils (110) in Position zu halten.

7. Vorrichtung zum Schutz des Knöchels einer Person (100) nach einem der vorhergehenden Ansprüche, bei der das starre Element (150) am hinteren Teil (111) des Hauptteils (110) über höhenverstellbare Befestigungsmittel befestigt ist, die ein erstes Element (153), das sich auf dem oberen Teil (151) des starren Elements (150) befindet, und ein zweites Element (154), das sich auf dem hinteren Teil (111) des Hauptteils (110) befindet, umfassen, so dass das erste (153) und das zweite (154) Element miteinander zusammenwirken werden, um das erste Element (153) auf dem zweiten Element (154) in mehreren Positionen zu halten.

8. Vorrichtung zum Schutz des Knöchels einer Person (100) nach den Ansprüchen 6 und 7, bei der das erste Element (153) ein Langloch ist und das zweite Element (154) aus Gewindeeinsätzen besteht, die am hinteren Teil (111) des Hauptteils (110) durch das hochdichte Verstärkungsteil (160) gehalten werden, um Schrauben (156) aufzunehmen, wobei das Langloch es ermöglicht, die Schrauben (156) durchzulassen und eine Anpassung an die gewünschte Höhe zu gewährleisten.

9. Vorrichtung zum Schutz des Knöchels einer Person (100) nach einem der vorhergehenden Ansprüche, die außerdem einen Schaumstoff (170) umfasst, der an der Innenwand (113) des Hauptteils (110) positioniert ist und sich an die Form des Knöchels (200) der Person anpasst.

10. Vorrichtung zum Schutz des Knöchels einer Person (100) nach einem der vorhergehenden Ansprüche, die außerdem eine Socke (180) umfasst, die den Vorderfuß umhüllt, wobei die Socke (180) und der Hauptteil (110) jeweils mit Druckknöpfen (181, 116) versehen sind, um die Socke (180) an der Vorrichtung (100) zu befestigen, wobei die Druckknöpfe (181) der Socke (180) dazu bestimmt sind, mit den Druckknöpfen (116) des Hauptteils (110) zusammenzuwirken.

## Claims

1. Ankle protection device for a person (100) comprising:
- a main part (110) made of an anti-abrasion material enveloping the ankle (200) and having a rear part (111) placed on the rear of the ankle (200) at the level of the Achilles tendon of the person, a front part (112) placed on the front of the ankle (200) at the level of the instep of the person, an inner wall (113) intended to be in contact with the ankle (200) and an outer wall (114),
- two reinforcements (120) fixed to the inner wall (113) of the main part (110) at the level of the malleoli of the person's ankle (200),
- closing devices fixed to the outer wall (114) of the main part (110) to hold and tighten the said device (100) against the ankle (200),
- a rigid element (150) having two parts joined together by a bend, an upper part (151) fixed to the rear part (111) of the main part (110) and a lower part (152) intended to be positioned under the heel of the person's shoe.

2. Ankle protection device for a person (100) according to the preceding claim, in which the constituent material of the main part (110) is a greased leather.

3. Ankle protection device for a person (100) according to one of the preceding claims, for which the closing devices correspond to straps (130) and buckles (140) fixed on either side of the outer wall (114) of the main part (110).

4. Ankle protection device for a person (100) according to the preceding claim, in which the straps (130) are mainly made of leather and include an area of self-gripping material.

5. Ankle protection device for a person (100) according to one of the preceding claims, in which the reinforcements (120) are made of thermoplastic polyurethane, acrylonitrile butadiene styrene or polypropylene.

6. Ankle protection device for a person (100) according to one of the preceding claims, further comprising a high-density reinforcement plate (160) fixed to the inner wall (113) of the main part (110) enabling the two reinforcements (120) on the inner wall (113) of the main part (110) enabling the two reinforcements (120) to be held in position on the inner wall (113) of the main part (110).

7. Ankle protection device for a person (100) according to one of the preceding claims, in which the rigid element (150) is fixed to the rear part (111) of the main part (110) by means of height-adjustable fixing means comprising a first element (153) located on the upper part (151) of the rigid element (150) and a second element (154) located on the rear part (111) of the main part (110), so that the said first (153) and second (154) elements will cooperate with each other to hold the first element (153) on the second element (154) in several positions.

8. Ankle protection device for a person (100) according to claims 6 and 7, for which the first element (153) is an oblong hole and the second element (154) consists of threaded inserts held at the rear part (111) of the main part (110) by the high-density reinforcement part (160) in order to receive screws (156), the oblong hole allowing the screws (156) to pass through and ensuring adjustment to the desired height.

9. Ankle protection device for a person (100) according to one of the preceding claims, which also includes a foam (170) positioned on the inner wall (113) of the said main part (110), to fit the shape of the ankle (200) of the person.

10. Ankle protection device for a person (100) according to one of the preceding claims, further comprising a sock (180) enveloping the front of the foot, said sock (180) and the main part (110) each being provided with press studs (181, 116) for securing said sock (180) to said device (100), the press studs (181) of said sock (180) being intended to cooperate with the press studs (116) of the main part (110).
